# EUROPEAN PATENT SPECIFICATION

(11) **EP 1 504 778 B1**
(45) Date of publication and mention of the grant of the patent: **17.01.2007**
(21) Application number: 04254741.4
(22) Date of filing: 06.08.2004
(51) Int. Cl.: A61L 31/16, A61M 5/142

(54) **Implantable pump for the treatment of obesity**
Implantierbare Pumpe zur Behandlung von Fettleibigkeit
Pompe implantable pour le traitement de l'obésité

(30) Priority: 06.08.2003 US 492848 P
(43) Date of publication of application: 09.02.2005
(73) Proprietor: ETHICON ENDO-SURGERY, Cincinnati, Ohio 45242 (US)
(72) Inventor: Rohr, William L., Marshfield, MA 02050 (US); Freeman, Lynette, West Chester, OH 45069 (US); Beaupre, Jean Michael, Cincinatti, OH 45249 (US); McKenna, Robert H., Cincinatti, OH 45241 (US); Warren, Alison, Basking Ridge, NJ 07920 (US); Sox, Thomas E., Ambler, PA 19002 (US)
(74) Representative: Tunstall, Christopher Stephen

(56) References cited:
- WO-A-02/085428
- WO-A-03/004034
- US-A- 5 782 798
- US-B1- 6 231 847
- AL-ARABI A ET AL: "Synergistic action by neuropeptide Y (NPY) and norepinephrine (NE) on food intake, metabolic rate, and brown adipose tissue (BAT) causes remarkable weight loss in the obese (fa/fa) Zucker rat." BIOMEDICAL SCIENCES INSTRUMENTATION. 1997, vol. 33, 1997, pages 216-225, XP009042507 ISSN: 0067-8856
- JALOWIEC J E ET AL: "OPIOID MODULATION OF INGESTIVE BEHAVIOR" PHARMACOLOGY BIOCHEMISTRY AND BEHAVIOR, vol. 15, no. 3, 1981, pages 477-484, XP001204589 ISSN: 0091-3057
- EARL J R ET AL: "Proinflammatory effects of morphine in the rat adjuvant arthritis model" INTERNATIONAL JOURNAL OF TISSUE REACTIONS, vol. 16, no. 4, 1994, pages 163-170, XP009042530 ISSN: 0250-0868
- MCCORKLE F M ET AL: "Biogenic amines regulate avian immunity." POULTRY SCIENCE. JUL 1993, vol. 72, no. 7, July 1993 (1993-07), pages 1285-1288, XP009042531 ISSN: 0032-5791
- FREUDENBERGER D O ET AL: "Glucose metabolism in a kangaroo (Macropus robustus erubescens) and a similar size eutherian herbivore, the feral goat." COMPARATIVE BIOCHEMISTRY AND PHYSIOLOGY. COMPARATIVE PHYSIOLOGY. OCT 1993, vol. 106, no. 2, October 1993 (1993-10), pages 295-298, XP009042529
- SHAHRARA SHIVA ET AL: "Subtype specific downregulation of thyroid hormone receptor mRNA by beta-adrenoreceptor blockade in the myocardium" BIOLOGICAL AND PHARMACEUTICAL BULLETIN, vol. 23, no. 11, November 2000 (2000-11), pages 1303-1306, XP001204749 ISSN: 0918-6158

## Description

### FIELD OF THE INVENTION

The present invention relates, in general, to tools for the control of obesity and, more particularly, to an implantable device for the delivery of appetite suppressant drugs to the ileum.

### BACKGROUND OF THE INVENTION

Obesity is considered a major health problem with annual associated costs reaching $100 billion in the U.S. alone. Morbid obesity is a condition of obesity with the presence of a secondary debilitating progressive disease and is generally associated with a body mass index (BMI) above 40 kg/m². While the basic mechanism of obesity is simply an imbalance between caloric intake and burn rate, the underlying factors are varied and complex and conservative attempts at sustained weight loss with this population are almost always unsuccessful. Often, there are genetic and other biological influences that may override environmental causes. Consequently, obesity is a disease that eludes a simple treatment, with a recurrence rate above 90% for those who attempt to lose weight. Moreover, long-term results using conservative treatments for morbid obesity are generally unsuccessful and are typically associated with further loss of self-esteem with the regaining of weight. Hypertension, cardiovascular disease, diabetes, along with a host of other co-morbidities all make morbid obesity second only to smoking as a preventable cause of death.

Currently, over 350 compounds with ten different modes of action are being investigated for the treatment of obesity. A major class of weight control agents is drugs that act on the central nervous system (CNS) to suppress appetite. One major subclass of CNS appetite suppressant drugs interacts with cathecolaminergic receptors in the brainstem. These include controlled drugs such as amphetamine, phenmetrazine, and diethylproprion, and over-the-counter drugs such as phenylpropanolamine. Manizidol is another CNS active drug that, although not a catecholamine, activates the central nervous system. Each of these agents have potential for addiction and, at doses which effectively reduce appetite, *i*.*e*., suppress food intake by 20-30%, they induce significant CNS side effects, such as nervousness, loss of concentration, and insomnia.

Another major class of weight control agents is drugs that promote malabsorption of nutrients through suppression of digestive enzymes. One agent in this category is Acarbose, a bacterial inhibitor of amylase and brushborder glycosidases. Another is tetrahydrolipostatin, a fungal inhibitor of lipases. These agents work by preventing digestion of carbohydrates and/or fats, thus creating an effective reduction in the number of calories absorbed, despite continued consumption. One drawback is that virtually complete inhibition of the respective enzymes must be maintained throughout the digestive period, a situation that can be rarely achieved.

A third class of weight control agents is noncaloric, non-nutritive dietary substitutes, like saccharin, aspartame or sucralose, sugar substitutes, and sucrose polyester, a fat substitute. These agents, while not absorbed, provide a taste and/or texture like the nutrient for which they are substituted. The disadvantage of these substitutes is that persons develop a hyperphagia to compensate for the reduction of calories by the substitution. Thermogenic drugs are also sometimes used. The catecholamine drugs discussed above have some thermogenic activity, in addition to their suppression of appetite. Thyroid hormone is also commonly used.

Surgical devices have also been employed to control appetite. Intragastric balloons have been placed endoscopically according to the theory that they increase the amount of gastric distension and thus augment satiety responses. However, they, have been discontinued in the United States because, while they were not shown to be any better than restricted diets in promoting weight loss, their long term use was associated with severe side effects such as gastric ulceration and migration of the balloons into the small intestine resulting in intestinal obstructions. Such devices are still marketed in Europe. Biliopancreatic by-pass, gastric by-pass, and gastric partitioning (stapling) are also current procedures, but the long-term side effects have not yet been determined.

Sensors that detect the presence of specific nutrients are present through the entire length of the small intestine. These sensors can recognize the presence of simple sugars, fats and fatty acids, and amino acids and short peptides. These sensors provide neural feedback that has two physiological effects. First, the rate of gastric emptying is slowed, where feedback from the ileum serves as a brake on gastric emptying and intestinal motility. Second, the sense of satiety after consuming food is prolonged. Release of the hormone peptide YY (PYY) by the nutrient sensing cells is thought to be at least partially responsible for the ileal brake effect as is glucagon-like peptide 1 (GLP1).

Intubation studies in dogs and rats have shown that perfusion of lipids into the terminal ileum, in comparison to infusion in more proximal portions of the small intestine, has a much more pronounced effect on delaying gastric emptying and decreasing food consumption. Thus the potency of the sensors in triggering a braking response increases with distance from the stomach.

Most macronutrients are quickly absorbed in the portions of the intestine most proximal to the stomach. The presence of nutrients in the distal ileum indicates to the body that gastric emptying is occurring too rapidly, and unabsorbed nutrients are reaching the colon and hence being wasted from a nutritional standpoint. The prolongation of satiety reduces the inclination of the organism to resume eating before the previous meal has been fully digested.

A coated pill has been developed that targets the ileum for the delivery of sugars, fatty acids, polypeptides, and amino acids to artificially stimulate the body's ileal brake feedback system. A coating on the pill retains the integrity of the pill capsule until the ileum is reached, whereby the release of the pill's contents decreases intestinal motility and increases a patient's feelings of satiety. However, there is significant variation in the GI tracts of those who are combating obesity. Existing pills are not generally tailored to the specific needs and body types of patients and therefore, in some instances, may miss the desired ileal delivery point altogether. Sporadic or ineffective manipulation of the ileal brake feedback mechanism may make the control of obesity a difficult or futile process. Therefore, the need exists for a device for artificially stimulating the ileal brake feedback system that consistently targets desired portions of a patient's GI tract in a manner that may be directly tailored to the patient's needs. The present invention fulfills these and other needs, and addresses deficiencies in known systems and techniques.

### SUMMARY OF THE INVENTION

The invention features devices for the intestinal delivery of active agents to promote or induce satiety in a subject. In the present invention, an active agent formulation comprises nutrients and pharmacological agents. The nutrients are generally selected from the group consisting of foodstuffs, amino acids, peptides, proteins, lipids, carbohydrates, vitamins and minerals. The active agent formulation is stored within an active agent delivery system (e.g., contained in a reservoir within the controlled active agent delivery system). The active agent formulation comprises an amount of active agent sufficient for treatment and is stable at body temperatures (i.e., no unacceptable degradation) for the entire pre-selected treatment period. The active agent delivery systems store the active agent formulation safely (e.g., without dose dumping), provide sufficient protection from bodily processes to prevent unacceptable degradation of the formulation, and release the active agent formulation in a controlled fashion at a therapeutically effective rate to treat hunger or obesity. In use, the active agent delivery system is implanted in the subject's body at an implantation site, and the active agent formulation is released from the active agent delivery system to a delivery site within the gastrointestinal tract. Preferably, the delivery site is the small intestine. More preferably, the delivery site is the ileum. The delivery site may be the same as, near, or distant from the implantation site. Once released at the delivery site, the active agent formulation enters the small intestines to act on nutrient sensors to modulate the satiety response.

Accordingly, the present invention provides the use of a formulation comprising an active agent selected from the group consisting of nutrients and pharmacological agents in a therapeutically effective amount sufficient for inducing or promoting a feeling of satiety in a subject for a period of at least 3 days in the manufacture of an implantable device for modulating satiety in the subject by intestinal delivery of the formulation to a site of action in the subject, whereby the active agent is present at the site of action within the gastrointestinal tract in an amount sufficient to modulate satiety, wherein the device comprises: a controlled active agent delivery system adapted for complete implantation at an implantation site selected from the group consisting of a subcutaneous site, an intraperitoneal site, a subdermal site, an intramuscular site, and an intra-adipose tissue site in the subject; a pump and the formulation.

Additionally, there is disclosed a method of long-term, site-specific delivery of a composition comprising a pharmaceutically acceptable active agent which may include at least one active ingredient selected from the group consisting of food grade nutrients (natural foodstuffs), and a pharmaceutically acceptable delivery agent, in a manner directly to a length of the intestine. The composition generally comprises a pharmaceutically acceptable active agent which may include at least one active ingredient selected from the group consisting of food grade nutrients (natural foodstuffs), and a pharmaceutically acceptable delivery agent, formulated for release of the active ingredients in the ileum. Food grade nutrients may include but are not limited to sugars, free fatty acids, polypeptides, amino acids and suitable foods that are precursors thereto.

For example, there is disclosed a method of modulating appetite in a subject, the method comprising: implanting in a subject, at an implantation site, an active agent delivery system comprising a pump and a formulation, the formulation comprising an active agent selected from the group consisting of nutrients and pharmacological agents, wherein the formulation comprises a therapeutically effective amount of the active agent sufficient for inducing or promoting a feeling of satiety in the subject, and delivering the formulation from the active agent delivery system to the subject whereby the active agent enters the gastrointestinal system, whereby the active agent is present at the site of action within the gastrointestinal tract in an amount sufficient to modulate satiety. Additionally, the patient may wear the pump externally where the delivery means is directed to the patient's gastrointestinal tract. When located externally, the pump may permit the patient to self-administer the satiety agent at prescribed times or as indicated by the patient's needs or sensory feedback.

In one embodiment, the active agent delivery system is implanted at an implantation site selected from the group consisting of a subcutaneous site, a subdermal site, an intramuscular site, and an intra-adipose tissue site.

In another embodiment, the formulation is delivered at a volume rate of from about 0.01 microliters per day to about 30 milliliters per day, In another embodiment, the delivering of the formulation is substantially continuous. In another embodiment, the active agent delivery system is coupled to a proximal end of a catheter for delivery of the formulation to a delivery site at a distance from the implantation site. In another embodiment, the pump is selected from the group consisting of an electromechanical pump, an electroosmotic pump, a hydrolytic pump, a piezoelectric pump, an elastomeric pump, a vapor pressure pump, a gravity feed pump, and an electrolytic pump. Additionally, a programmable rate pump is contemplated having a clock as an active element wherein different infusion rates are administered at different times of the day. Varying infusion rates may provide patients with a more natural appetite at pre-determined periods established in accordance with circadian rhythms.

In another embodiment, the formulation comprises nutrients selected from the group consisting of foodstuffs, amino acids, peptides, proteins, lipids, carbohydrates, vitamins and minerals.

According to one embodiment of the invention, the active ingredient is selected from the group consisting of sugars, fatty acids, phenylalanine polypeptides, and amino acids. According to another embodiment, the active ingredient may include monomeric sugars, such as glucose and xylose. Furthermore, chemical derivatives or chemical analogs of natural foodstuffs may be used in place of, or together with, natural foodstuffs to enhance the potency of the satiety response, through more favorable solubility, buffered pH absorption, affinity to nutrient sensors in the intestine, or some combination of these properties.

There is further disclosed a method for controlling appetite comprising long-term delivery of selected active agents to specific portions of the gastrointestinal tract at a controlled delivery rate. The gastrointestinal tract is about 25 to 30 feet long in the adult and includes the mouth, esophagus, stomach, small intestine (duodenum, jejunum and ileum), and large intestine (caecum, colon, and rectum) and anal canal.

There is also disclosed a method for controlling appetite comprising spreading a selected active agent over a length of intestine. The method may further comprise delivering the active ingredient predominantly in the ileum. Once release begins, preferably it occurs over the length of the ileum.

The above summary is not intended to describe each embodiment or every implementation of the present invention. Advantages and attainments, together with a more complete understanding of the invention, will become apparent and appreciated by referring to the following detailed description and claims taken in conjunction with the accompanying drawings.

Further aspects and advantages of this invention will be disclosed in the following examples, which should be regarded as illustrative and not limiting the scope of this application.

Throughout this document, all temperatures are given in degrees Celsius, and all percentages are weight percentages unless otherwise stated. The following are definitions of terms used in this specification. The initial definition provided for a group or term herein applies to that group or term throughout the present specification, individually or as part of another group, unless otherwise indicated.

### BRIEF DESCRIPTION OF THE DRAWINGS

This invention, as defined in the claims, can be better understood with reference to the following drawings. The drawings are not necessarily to scale, emphasis instead being placed upon clearly illustrating principles of the present invention.

The novel features of the invention are set forth with particularity in the appended claims. The invention itself, however, both as to organization and methods of operation, together with further objects and advantages thereof, may best be understood by reference to the following description, taken in conjunction with the accompanying drawings in which:

Figure 1 is a pictorial view of a active agent reservoir and pump in accordance with the present invention;

Figure 2 is a pictorial view of a active agent delivery catheter in accordance with the present invention;

Figure 3 is a side view of the active agent delivery catheter disposed within the ileum and connected to the active agent reservoir and pump located in the subcutaneous fatty tissue of the human body; and

Figure 4 is flow diagram of a method of providing a satiety agent to the gastrointestinal tract of a patient.

In the following description of the illustrated embodiments, references are made to the accompanying drawings, which form a part hereof, and in which is shown by way of illustration various embodiments in which the invention may be practiced. It is to be understood that other embodiments may be utilized, and structural and functional changes may be made without departing from the scope of the present invention.

### DETAILED DESCRIPTION OF THE PREFERRED EMBODIMENT

Before the present invention for modulation of appetite and satiety is described, it is to be understood that this invention is not limited to the specific methodology, devices, therapeutic formulations, and appetite and obesity syndromes described as such may, of course, vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "and", and "the" include plural referents unless the context clearly dictates otherwise. Thus, for example, reference to "an active agent delivery system" includes a plurality of such devices and reference to "the method of delivery" includes reference to equivalent steps and methods known to those skilled in the art, and so forth.

Unless defined otherwise, all technical and scientific terms used herein have the same meaning as commonly understood to one of ordinary skill in the art to which this invention belongs. Although any devices and materials similar or equivalent to those described herein can be used in the practice or testing of the invention, the preferred devices and materials are now described.

All publications mentioned herein are for the purpose of describing and disclosing compositions and methodologies which might be used in connection with the presently described invention. The publications discussed herein are provided solely for their disclosure prior to the filing date of the present application. Nothing herein is to be construed as an admission that the invention is not entitled to antedate such a disclosure by virtue of prior invention.

The invention features devices for the intestinal delivery of active agents to promote or induce satiety in a subject. In the present invention, an active agent formulation comprising nutrients and pharmacological agents. The nutrients are generally selected from the group consisting of foodstuffs, amino acids, peptides, proteins, lipids, carbohydrates, vitamins and minerals. The active agent formulation is stored within an active agent delivery system (*e*.*g*., contained in a reservoir within the controlled active agent delivery system). The active agent formulation comprises an amount of active agent sufficient for treatment and is stable at body temperatures (*i*.*e*., no unacceptable degradation) for the entire pre-selected treatment period. The active agent delivery systems store the active agent formulation safely (*e*.*g*., without dose dumping), provide sufficient protection from bodily processes to prevent unacceptable degradation of the formulation, and release the active agent formulation in a controlled fashion at a therapeutically effective rate to treat hunger or obesity. In use, the active agent delivery system is implanted in the subject's body at an implantation site, and the active agent formulation is released from the active agent delivery system to a delivery site within the gastrointestinal tract. Preferably, the delivery site is the small intestines. More preferably, the delivery site is the ileum. The delivery site may be the same as, near, or distant from the implantation site. Once released at the delivery site, the active agent formulation enters the small intestines to act on intestinal sensors to modulate the satiety response.

The absorption of fats or lipids (terms used herein interchangeably) occurs mainly upon their entry into the small intestine. In the small intestine, specific receptors for fats and proteins, and the osmolality, acidity and the particle size of the meal activate propulsive and inhibitory reactions (*i*.*e*., ileal braking), which then modulate their transit and absorption. The present invention features devices ideally suited for the intestinal delivery of active agents for the treatment of satiety by promoting ileal braking in a subject.

The term "satiety" is used here to generally describe the any state of being satiated or some fullness of appetite, that is, some increased amount of the satisfied feeling of being full after eating. Satiety can be measured either in terms of the amount of food consumed (meal size), latency to rest or by the duration between consumption of food (intermeal interval). Early satiety is having the feeling of being full prematurely (sooner than normal or after eating less than usual). The physiological process that stops eating is unconscious and is called satiation. It normally begins early in a meal and terminates when eating ends. As used here, satiety is a process that occurs during a meal or postprandial satiety, a state of noneating that begins at the end of one meal and lasts until the beginning of the next meal.

The term "satiety modulation or treatment" is used here to generally describe regulation, regression, suppression, or mitigation of appetite so as to make the subject more comfortable as determined by subjective criteria, objective criteria, or both. Preferably, the methods and devices herein are used to create early satiety in a subject but do not require complete satiety, only some increase in the satiety response. In general, satiety is assessed subjectively by patient report, with the health professional taking into consideration the patient's age, cultural background, environment, and other psychological background factors known to alter a person's subjective reaction to food and hunger.

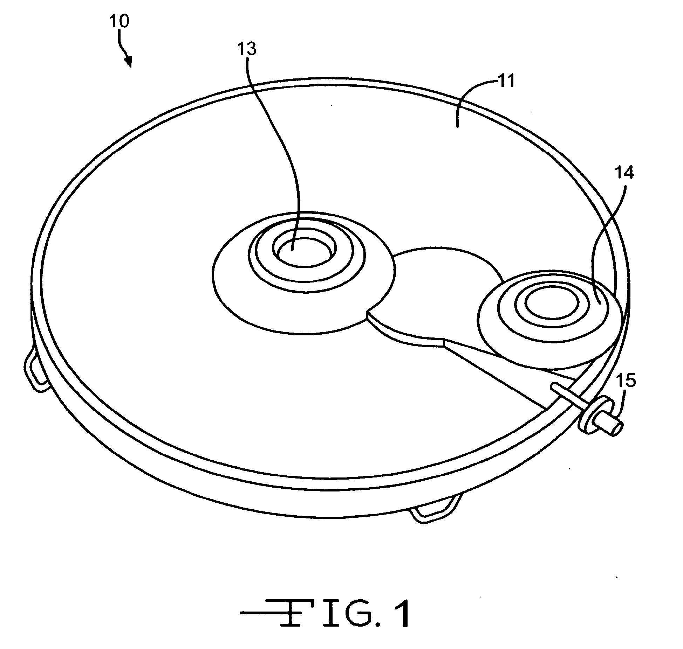

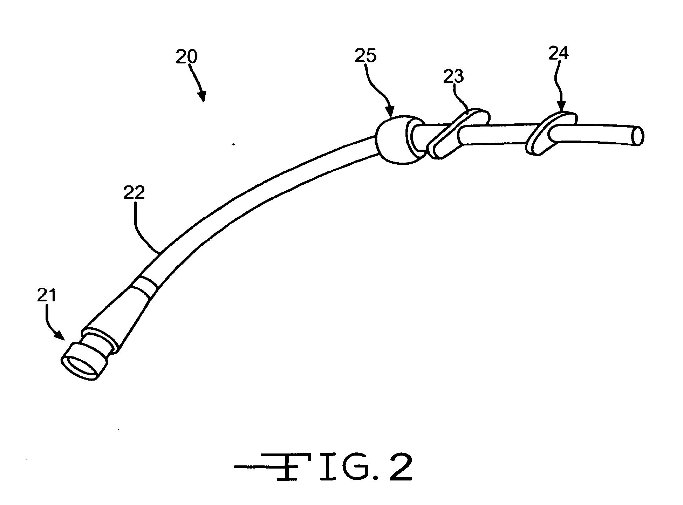

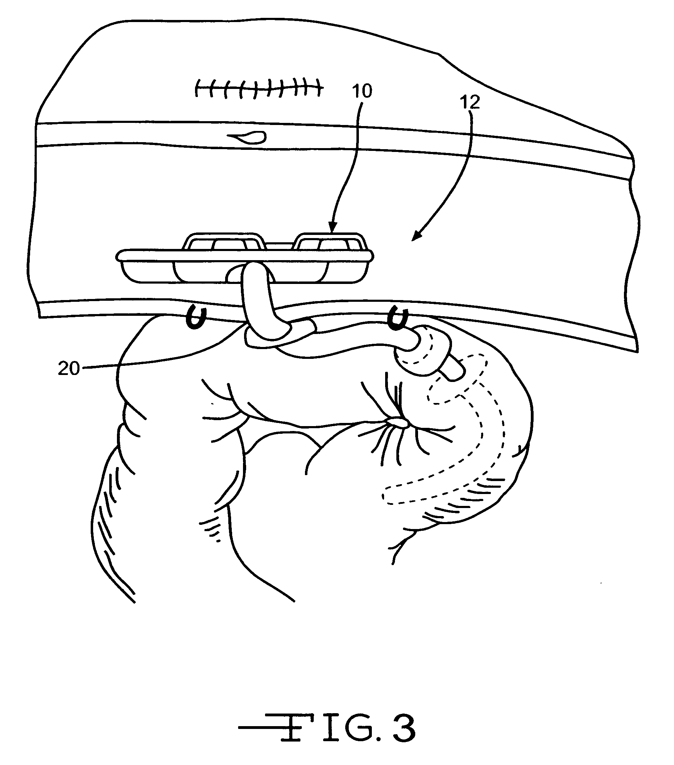

Referring now to Figs. 1,2, and 3, the present invention comprises an active agent catheter delivery system 12 having an active agent reservoir and pump 10 and an active agent delivery catheter 20. The reservoir and pump 10 may be any suitable reservoir and/or fluid delivery pump having, for example, a resealable fluid insertion boss 13, a fluid reservoir 11, a fluid pump 14, and a radially extended male fluid delivery port as further discussed herein. Insertion boss 13 may be constructed from any suitable material such as, for example, polyolefins (high density polyethylene (HDPE), low density polyethylene (LDPE), linear low density polyethylene (LLDPE), polypropylene (PP), and the like), that permits a syringe or other fluid delivery means to be passed through insertion boss into a cavity (not shown) of fluid reservoir 11 for the delivery of an active agent therein. Upon removal of the fluid delivery means, the material comprising insertion boss 13 will substantially reseal, thereby preventing the escape of the active agent.

Active agent delivery catheter 20 may be any suitable fluid delivery means such as, for example, a jejunostomy tube. Catheter 20 includes a female port 21 positioned at a first terminal end of catheter 20 and adapted to mate with the male fluid delivery port 15 of reservoir and pump 10. Catheter 20 includes an elongate fluid transmission lumen 22 extending from female port 21 to the second terminal end of catheter 20. Positioned near the second terminal end of the catheter 20 and around the circumference of lumen 22 is a first laterally extending brace 23. A second laterally extending brace 24 is positioned distally to first laterally extending brace 23 in close proximity to the second terminal end of catheter 20.

Pump and reservoir 10 is adapted for placement subcutaneously within the fatty layers of the human body, intra-abdominally, intra-lumenally, or in any other suitable position as will be further discussed herein. The female port 21 of catheter 20 is adapted to fluidly mate with the male fluid delivery port 15 permitting fluid from reservoir 11 to be pumped through lumen 22 of catheter 20. Catheter 20 is adapted for subcutaneous placement, wherein the first laterally extending brace 23 is anchored to the fascia of the abdominal wall in the extra-peritoneal space. Additionally, a plurality of braces may be employed to provide a secure attachment to, for example, Scarpa's fascia to provide a seal resistant to slippage and bacterial infection. First laterally extending brace 23 prevents catheter 20 from moving undesirably once inserted. Second laterally extending brace 24 and the distal portion of catheter 20 are adapted for placement within a patient's ileum, wherein second laterally extending brace 24 secures catheter 20 within the ileum. Active agent catheter delivery system 12 provides for a remote delivery of active agent from an implant site to a delivery site within the lumen of the intestines. Catheter 20 further includes a balloon 25 adapted to secure catheter 20 within the abdominal cavity wherein a securing means, such as a row of purse-string sutures, may be placed and tightened around the opening in the intestine to secure the intestine to catheter 20. The balloon 25 may then be pulled taut against the sealing means to prevent leakage of intestinal contents. Additionally, the catheter system includes a bacteriologic filter, an impregnated antibiotic, and/or a one way valve to prevent retrograde spread of the body's normal bacterial flora into the pump.

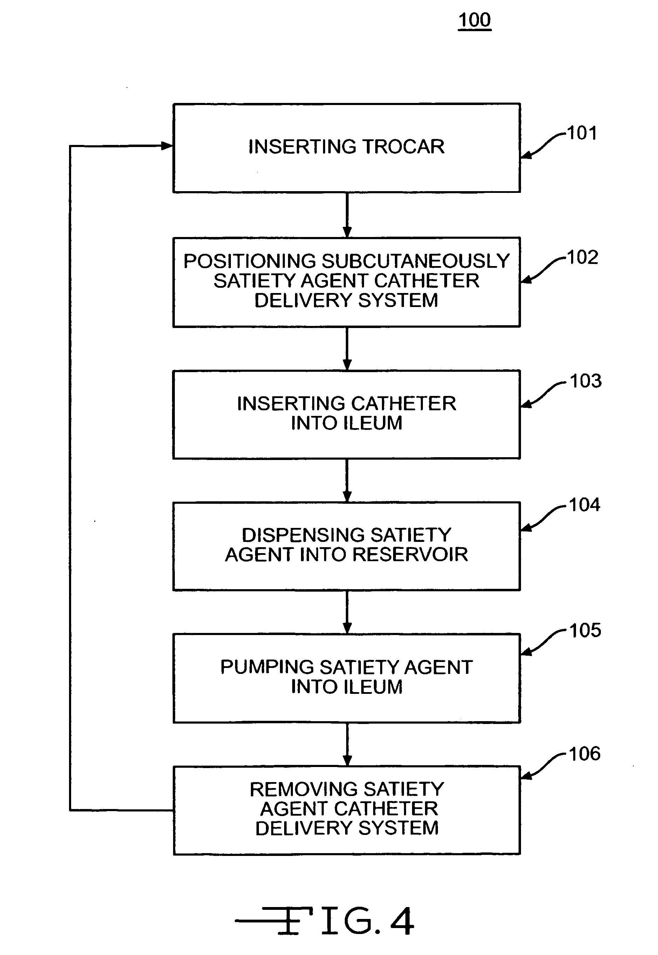

Referring to Figure 4, Step 101 of Method 100 comprises inserting a trocar or other suitable insertion means into the abdominal cavity as is commonly known in the art. Step 102 of Method 100 comprises subcutaneously inserting active agent catheter delivery system 12 into a patient's body, wherein reservoir and pump 10 may be positioned within the fatty tissue surrounding the abdominal cavity and the proximal portion of the catheter 20 may pass into the abdominal cavity. Step 102 further comprises passing first laterally extending brace 23 into the cavity between the abdominal wall and ileum and passing balloon 25 into the ileum wherein a securing means, such as a row of purse-string sutures, may be placed and tightened around the opening in the intestine to secure the intestine to catheter 20. The balloon 25 may then be pulled taut against the sealing means to prevent leakage of intestinal contents.

Step 103 of Method 100 comprises inserting the second terminal end of catheter 20 into the ileum of the patient, wherein the first laterally extending brace remains within the abdominal cavity and the second laterally extending brace passes into the ileum. Active agent catheter delivery system 12 is directed to the ileum in the preferred embodiment of the present invention, however, the present invention further comprises positioning active agent catheter delivery system 12 at additional locations as further discusses herein.

Step 104 of Method 100 comprises inserting a syringe or other suitable fluid delivery means through the dermal layers and into the fluid insertion boss 13 of reservoir and pump 10 as will be further discussed herein. Step 105 of Method 100 comprises dispensing an active agent from reservoir 11, through catheter 20, into the ileum of the patient into order to decrease intestinal motility and increase feelings of satiety experienced by the patient. The active agent may be dispensed at any suitable interval at any suitable dosage for the treatment of obesity. Optionally, the reservoir 11 may be recharged at any time necessary. Preferably, recharging of the reservoir 11 is performed without removal from the implantation site but is performed remotely such as, for example, by injection with a syringe. Additionally, the present invention comprises a multi-chambered or multi-reservoir pump combined with a multi-lumen catheter, whereby active agents having a short half-life can be blended just prior to infusion. Furthermore, this allows for differential delivery rates from each of the plurality of chambers to be tailored to the specific needs of the patient.

Preferably, the reservoir 11 is recharged no more than every 7 days. More preferably, the reservoir 11 is recharged no more than every 30 days. Step 106 of Method 100 comprises removing active agent catheter delivery system 12 when treatment for the condition of obesity is complete. Active agent catheter delivery system 12 may be removed by trocar or by any other suitable means commonly known in the art.

Illustrations of method steps, such as, for example, the steps illustrated in Figure 4, show steps sequentially and in a particular order. There is no need to perform the steps in the order illustrated. Deviating from the illustrated order for some or all of the steps is contemplated by the invention.

Alternative features may be used.

The present system is designed to maximize satiety feedback from normal intestinal sensors by small amounts of nutrients or nutrient derivatives, in essence, to "fool" body sensors that are not usually in contact with nutrients unless very large amounts are ingested. Significant advantages of this approach are the likelihood of minimal or no toxicity as well as increased patient compliance due the absence of active regimen steps required by the patient.

In particular, a method is disclosed for manipulating the sense of satiety developed from the gastrointestinal transit of a substance in a mammal, whether the substance be a food or drug compound. The method involves administering a therapeutically effective amount, by a direct delivery route, of a pharmaceutically acceptable composition comprising an active agent to the mammal's gastrointestinal tract. As used herein, the term, "active agent" includes, without limitation, any substance that it is desired to incorporate into a delivery system for sustained or controlled delivery and/or release and is capable of inducing or promoting a feeling of satiety in a subject. An active agent can be in any state, including liquids, solutions, pastes, solids, and the like. The active agent may be a pharmaceutically active agent, such as an active agent and/or diagnostic substance for human or veterinary use. An active agent may also be, simply by way of example, any art known agent, *e*.*g*., a polypeptide or peptide derivative effective to induce a feeling of satiety. By the term "administer" is intended to mean introducing the delivery system or device of the present invention into a subject. When administration is for the purpose of treatment, administration may be for either prophylactic or therapeutic purposes. When provided prophylactically, the substance is provided in advance of any symptom. The prophylactic administration of the substance serves to prevent or attenuate any subsequent symptom. When provided therapeutically, the substance is provided at (or shortly after) the onset of a symptom. The therapeutic administration of this substance serves to attenuate any actual symptom. The term "subject" is meant any subject, generally a mammal (*e*.*g*., human, canine, feline, equine, bovine, *etc*.), in which management of appetite or obesity is desired. The term "therapeutically effective amount" is meant an amount of a therapeutic agent, or a rate of delivery of a therapeutic agent, effective to facilitate a desired therapeutic effect. The precise desired therapeutic effect (*e*.*g*., the degree of satiety) will vary according to the condition to be treated, the formulation to be administered, and a variety of other factors that are appreciated by those of ordinary skill in the art. In general, the invention involves the suppression or mitigation of hunger in a subject that may be associated with any of a variety of identifiable or unidentifiable etiologies. Additionally, it is further contemplated that the delivery system of the present invention be used in the treatment of, for example, Crohn's disease, or other conditions that may benefit from the intra-abdominal delivery of drugs as well as the provision of nutrient supplements in cancer patients. It is further contemplated that the system of the present invention be used in combination with a strict dietary regimen to give patients a chance at successful rehabilitation once the system is removed.

Since satiety feedback from distal small bowel (ileum) is more intense per amount of sensed active agent (*e*.*g*., nutrients) than from proximal bowel (jejunum), delivering the nutrients predominately in the ileum will also enhance the satiety response per amount of agent delivered. Thus, both the spread and predominant site of delivery (ileum) will maximize the effect, so that a small amount of released nutrient will be sensed as though it were a large amount, creating a high satiating effect.

The active agent is selected from the group consisting of nutrients and pharmacological agents. The nutrients are generally selected from the group consisting of foodstuffs, amino acids, peptides, proteins, lipids, carbohydrates, vitamins and minerals.

Suitable protein sources include milk, soy, rice, meat (*e*.*g*., beet, animal and vegetable (*e*.*g*., pea, potato), egg (egg albumen), gelatin, and fish. Suitable intact proteins include, but are not limited to, soy based, milk based, casein protein, whey protein, rice protein, beef collagen, pea protein, potato protein and mixtures thereof. Suitable protein hydrolysates also include, but are not limited to, soy protein hydrolysate, casein protein hydrolysate, whey protein hydrolysate, rice protein hydrolysate, potato protein hydrolysate, fish protein hydrolysate, egg albumen hydrolysate, gelatin protein hydrolysate, a combination of animal and vegetable protein hydrolysates, and mixtures thereof. Hydrolyzed proteins (protein hydrolysates) are proteins that have been hydrolyzed or broken down into shorter peptide fragments and amino acids.

In the broadest sense, a protein has been hydrolyzed when one or more amide bonds have been broken. Breaking of amide bonds may occur unintentionally or incidentally during manufacture, for example due to heating or shear. For purposes of this invention, the term hydrolyzed protein means a protein that has been processed or treated in a manner intended to break amide bonds. Intentional hydrolysis may be effected, for example, by treating an intact protein with enzymes or acids.

Favored proteins include extensively hydrolyzed protein hydrolysates prepared from acid or enzyme treated animal and vegetable proteins, such as, casein hydrolysate, whey hydrolysate, casein/whey hydrolysate, soy hydrolysate, and mixtures thereof. By "extensively hydrolyzed" protein hydrolysates it is meant that the intact protein is hydrolyzed into peptide fragments whereby a majority of peptides fragments have a molecular weight of less than 1000 Daltons. More preferably, from at least about 75% (preferably at least about 95%) of the peptide fragments have a molecular weight of less than about 1000 Daltons.

The amino acids may be one or more of aspartic acid, alanine, arginine, asparagine, cysteine, glycine, glutamic acid, glutamine, histidine, isoleucine, leucine, lysine, methionine, phenylalanine, proline, serine, threonine, tryptophan, tyrosine, and valine. Preferred amino acids are L-phenylalanine, L-tryptophan, L-tyrosine, L-cystine, L-taurine, L-methionine, L-arginine, and L-carnitine. More preferred amino acids are L-phenylalanine and L-tryptophan.

Suitable foodstuffs, amino acids, peptides, proteins lipids, carbohydrates, vitamins and minerals can vary widely and are well known to those skilled in the art of making pediatric formulas. Carbohydrates useful in the present invention include mono-, di- and polysaccharides. Preferred saccharides include, *e*.*g*., glucose, fructose, mannose, galactose, sucrose, maltose, lactose, maltodextrins and glucose polymers. Preferably, the carbohydrate is maltose. Suitable carbohydrates may thus include, but are not limited to, hydrolyzed, intact, naturally and/or chemically modified starches sourced from corn, tapioca, rice or potato in waxy or non waxy forms; and sugars.

Suitable vitamins include, but are not limited to, vitamins A, E, C, D, K, the B complex vitamins, pantothenic acid, thiamin, niacin, niacinamide, riboflavin, iron and biotin. Minerals include, but are not limited to, calcium, chromium, phosphorus, sodium, chloride, magnesium, manganese, iron, copper, zinc, selenium, and iodine. Salts may also be used. Suitable salts include, but are not limited to, sodium, potassium, magnesium and calcium.

Suitable lipids include, but are not limited to, coconut oil, soy oil, corn oil, olive oil, safflower oil, high oleic safflower oil, MCT oil (medium chain triglycerides), sunflower oil, high oleic sunflower oil, palm oil, palm olein, canola oil, lipid sources of arachidonic acid and docosahexaneoic acid, and mixtures thereof. Lipid sources of arachidonic acid and docosahexaneoic acid include, but are not limited to, marine oil, egg yolk oil, and fungal oil.

Depending on the desired results, the active agent can include an active lipid; a serotonin, serotonin agonist, or serotonin re-uptake inhibitor; peptide YY or a peptide YY functional analog; calcitonin gene-related peptide (CGRP) or a CGRP functional analog; an adrenergic agonist; an opioid agonist; a combination of any of any of these; or an antagonist of a serotonin receptor, peptide YY receptor, CGRP receptor; adrenoceptor and/or opioid receptor; and/or glucagon-like peptide 1 (GLP1).

Preferably, in order to induce a feeling of satiety in the subject, the active agent is one or more agents selected from the group of an active lipid; a serotonin, serotonin agonist, or serotonin re-uptake inhibitor; peptide YY or a peptide YY functional analog; GLP1 peptides and GLP1 analogs, calcitonin gene-related peptide or a functional analog; CGRP or a CGRP functional analog; an adrenergic agonist; an opioid agonist; or a combination of any of these, which is delivered in an amount and under conditions such that the cholinergic intestino-fugal pathway, at least one prevertebral ganglionic pathway, the adrenergic efferent neural pathway, the serotonergic interneuron and/or the opioid interneuron are activated thereby.

Serotonin, or 5-hydroxytryptamine (5-HT) is preferably used at a dose of 0.005-0.75 mg/kg of body mass. Serotonin re-uptake inhibitors include Prozac or Zoloft.

Serotonin receptor antagonists include antagonists of 5-HT3, 5-HT1P, 5-HT1A, 5-HT2, and/or 5-HT4 receptors. Examples include ondansetron or granisetron, 5HT3 receptor antagonists (preferred dose range of 0.04-5 mg/kg), deramciclane, or alosetron. 5-HT4 receptor antagonists are preferably used at a dose of 0.05-500 picomoles/kg.

Peptide YY (PYY) and its functional analogs are preferably delivered at a dose of 0.5-500 picomoles/kg. PYY functional analogs include PYY (22-36), BIM-43004 (Liu, C D, *et al*., J. Surg. Res. 59(1):80-84 [1995]), BIM-43073D, BIM-43004C (Litvak, D. A. *et al*., Dig. Dis. Sci. 44(3):643-48 [1999]). Other examples are also known in the art (*e*.*g*., U.S. Pat. No. 5,604,203). PYY receptor antagonists preferably include antagonists of Y4/PP1, Y5 or Y5JPP2/Y2, and most preferably Y1 or Y2. (*e*.*g*., U.S. Pat. No. 5,912,227) Other examples include BIBP3226, CGP71683A (King, P. J. *et al*., J. Neurochem. 73(2):641-46 [1999]).

Adrenergic agonists include norepinephrine. Adrenergic or adrenoceptor antagonists include β-adrenoceptor antagonists, including propranolol and atenolol. They are preferably used at a dose of 0.05-2 mg/kg.

Opioid agonists include delta-acting opioid agonists (preferred dose range is 0.05-50 mg/kg, most preferred is 0.05-25 mg/kg); κ-acting opioid agonists (preferred dose range is 0.005-100 microgram/kg); µ-acting opioid agonists (preferred dose range is 0.05-25 µg/kg); and episilon-acting agonists.

Opioid receptor antagonists include µ-acting opioid antagonists (preferably used at a dose range of 0.05-5 microgram/kg); κ-opioid receptor antagonists (preferably used at a dose of 0.05-30 mg/kg); Δ opioid receptor antagonists (preferably used at a dose of 0.05-200 microgram/kg); and ε-opioid receptor antagonists. Examples of useful opioid receptor antagonists include naloxone, naltrexone, methylnaltrexone, nalmefene, H2186, H3116, or fedotozine, *i*.*e*., (+)-1-1 [3,4,5-trimethoxy)benzyloxymethyl]-1-phenyl-N,N-dimethylpropylamine. Other useful opioid receptor antagonists are known (*e*.*g*., U.S. Pat. No. 4,987,136).

In one embodiment, the active agent is one or more active lipid. Preferably, the active lipid is selected from the group consisting of saturated and unsaturated fatty acids, fully hydrolyzed fats and mixtures thereof, in an amount and in a form effective to promote contact of the lipid with the subject's small intestine and, thereby, inducing satiety.

The active lipid can be in the form of a composition that upon delivery to the intestine releases the active lipid into the proximal segment of the small intestine, so as to prolong the residence time of the substance in the small intestine and, thereby, increase, dissolution, bioavailability and satiety effect

As used herein, "active lipid" encompasses a digested or substantially digested molecule having a structure and function substantially similar to a hydrolyzed end-product of fat digestion. Examples of hydrolyzed end products are molecules such as diglyceride, monoglyceride, glycerol, and most preferably free fatty acids or salts thereof.

In a preferred embodiment, the active agent is an active lipid comprising a saturated or unsaturated fatty acid. Fatty acids contemplated by the invention include fatty acids having between 4 and 24 carbon atoms.

Examples of fatty acids contemplated for use in the practice of the present invention include caprolic acid, caprulic acid, capric acid, lauric acid, myristic acid, oleic acid, palmitic acid, stearic acid, palmitoleic acid, linoleic acid, linolenic acid, trans-hexadecanoic acid; elaidic acid, columbinic acid, arachidic acid, behenic acid eicosenoic acid, erucic acid, bressidic acid, cetoleic acid, nervonic acid, Mead acid, arachidonic acid, timnodonic acid, clupanodonic acid, docosahexaenoic acid, and the like. In a preferred embodiment, the active lipid comprises one or more of oleic acid, dodecanoic acid and glycerol monooleate.

Also preferred are active lipids in the form of pharmaceutically acceptable salts of hydrolyzed fats, including salts of fatty acids. Sodium or potassium salts are preferred, but salts formed with other pharmaceutically acceptable cations are also useful. Useful examples include sodium- or potassium salts of caprolate, caprulate, caprate, laurate, myristate, oleate, palmitate, stearate, palmitolate, linolate, linolenate, trans-hexadecanoate, elaidate, columbinate, arachidate, behenate, eicosenoate, erucate, bressidate, cetoleate, nervonate, arachidonate, timnodonate, clupanodonate, docosahexaenoate, and the like. In a preferred embodiment, the active lipid comprises an oleate and/or dodecanate salt.

Sodium dodecanoate or sodium dodecylsulfate are the preferred active ingredients. Since the availability of pancreatic enzymes to digest polymeric nutrients in the ileum is unpredictable, the nutrient is best delivered in a predigested (monomeric) form. It has been shown that glucose is not a good active agent in the ileum but that fat does act well. Furthermore, fatty acids, like dodecanoic acid, are much more slowly absorbed than glucose, so that even small amounts may achieve a long length of contact.

Another preferred active ingredient is sodium dodecylsulfate. This material is known to be biologically active in the proximal intestinal lumen where it stimulates pancreatic secretion even better than dodecanoic acid. Sodium dodecanoate and sodium dodecylsulfate are much more readily soluble at luminal pH than sodium oleate. The dodecanoate does not require bile salt to emulsify it, whereas dispersion of sodium oleate would be aided by bile salt. Dispersion into solution is necessary for the nutrient to contact the sensory nerves in the intestinal mucosa. Including an incipient, such as NaCl or trisodium citrate may increase the density of the delivered nutrients.

The active agents suitable for use with this invention may be employed in dispersed form in a pharmaceutically acceptable carrier. As used herein, useful carrier is a commercially available emulsion, ENSURE, but active lipids, such as oleate or oleic acid are also dispersible in gravies, dressings, sauces or other comestible carriers. Dispersion can be accomplished in various ways, preferably as a solution.

Lipids can be held in solution if the solution has the properties of bile (*i*.*e*., solution of mixed micelles with bile salt added), or the solution has the properties of a detergent (*e*.*g*., pH 9.6 carbonate buffer) or a solvent (*e*.*g*., solution of Tween^{™}). The second is an emulsion which is a 2-phase system in which one liquid is dispersed in the form of small globules throughout another liquid that is immiscible with the first liquid. The third is a suspension with dispersed solids (*e*.*g*., microcrystalline suspension). Additionally, any emulsifying and suspending agent that is acceptable for human consumption can be used as a vehicle for dispersion of the composition. For example, gum acacia, agar, sodium alginate, bentonite, carbomer, carboxymethylcellulose, carrageenan, powdered cellulose, cholesterol, gelatin, hydroxyethyl cellulose, hydroxypropyl cellulose, hydroxypropyl methylcellulose, methylcellulose, octoxynol 9, oleyl alcohol, polyvinyl alcohol, povidone, propylene glycol monostearate, sodium lauryl sulfate, sorbitan esters, stearyl alcohol, tragacanth, xantham gum, chondrus, glycerin, trolamine, coconut oil, propylene glycol, malt, and malt extract.

Other preferred active agents include lipotrophic vitamins. Lipotropics can increase the mobilization of fatty acids, thus helping people to lose body fat. These include choline, methionine, folate and Vitamin B 12 and are important for lipid metabolism and synthesis of cellular membranes. The active agent can include conjugated linoleic acid. Conjugated linoleic acid, referred to as "CLA" is a mixture of one or all of the isomers of octadecadienoic acid including the cis-9, trans-11; cis-9, cis-11; trans-9, cis-11; trans-9, trans-11; cis-10-cis-12; cis-10, trans-12; trans-10, cis-12; and trans-10, trans-12 cis-9, trans-1 1 and trans-10, cis-12 isomers are cis-11; trans-9, trans-11; cis-10-cis-12; cis-10, trans-12; trans-10, cis-12; and trans-10, trans-12 cis-9, trans-11 and trans-10, cis-12 isomers are thought to have the most biological activity. Studies in animals suggest that CLA enhances lipolysis, the breakdown of fat.

Other pharmacological agents that can be added to the active agent include somatostatin analogues, insulin release inhibitors, anti-diarrheal agents, antibiotics, fiber and electrolytes.

Any of these formulations can be incorporated into a solution, emulsion or suspension containing the active agent. Active agents can be provided in any of a variety of formulations compatible with intestinal delivery, provided that such formulation is stable (*i*.*e*., not subject to degradation to an unacceptable amount at body temperature). The concentration of active agents in the formulation may vary from about 0.1 wt. % to about 80 or even 100 wt %. The active agents can be provided in any form suitable to be carried by the controlled drug delivery device and released for distribution, *e*.*g*., solid, semisolid, gel, liquid, suspension, emulsion, osmotic dosage formulation, diffusion dosage formulation, erodible formulation, *etc*. Of particular interest is the administration of active agents in a form suitable for administration using an implanted pump such as, for example, an osmotic pump.

Pharmaceutical grade organic or inorganic carriers and/or diluents suitable for systemic delivery can be included in the formulations suitable for delivery according to the invention. Such physiologically acceptable carriers are well known in the art. Exemplary liquid carriers for use in accordance with the present invention can be sterile non-aqueous or aqueous solutions that contain no materials other than the active ingredient. The formulations can optionally further comprise buffers such as sodium phosphate at physiological pH value, physiological saline or both (*i.e.*, phosphate-buffered saline). Suitable aqueous carriers may

In one embodiment, the formulation comprises active agents and a nonionic surfactant. Suitable nonionic surfactants include those which are pharmaceutically acceptable, including but not limited to, polysorbate, *e*.*g*., polysorbate 20, polysorbate 40, polysorbate 60; sorbitan trioleate; polyoxyethylene polyoxypropyleneglycol, *e*.*g*., polyoxyethylene(160)glycol, and polyoxypropylene(30)glycol. Other nonionic surfactants which are suitable for use in the formulations include nonionic surfactants of the fatty acid polyhydroxy alcohol ester type such as sorbitan monolaurate, monooleate, monostearate or monopalmitate, sorbitan tristearate or trioleate, adducts of polyoxyethylene and fatty acid polyhydroxy alcohol esters such as polyoxyethylene sorbitan monolaurate, monooleate, monostearate, monopalmitate, tristearate or trioleate, polyethylene glycol fatty acid esters such as polyoxyethyl stearate, polyethylene glycol 400 stearate, polyethylene glycol 2000 stearate, in particular ethylene oxide-propylene oxide block copolymers of the Pluronics^{™} (Wyandotte) or Synperonic^{™} (ICI). In particular embodiments, the nonionic surfactant is polysorbate 20, polysorbate 40, polysorbate 60, or sorbitan trioleate, or mixtures of one or more of the foregoing.

The formulations comprising active agents and suitable for administration according to the invention may comprise additional active or inert components that are pharmaceutically acceptable and compatible with the active ingredient. Suitable excipients can comprise dextrose, glycerol, alcohol (*e*.*g*., ethanol), and the like, and combinations of one or more thereof with vegetable oils, propylene glycol, polyethylene glycol, benzyl alcohol, benzyl benzoate, dimethyl sulfoxide (DMSO), organics, and the like to provide a suitable composition. In addition, if desired, the composition can comprise hydrophobic or aqueous surfactants, dispersing agents, wetting or emulsifying agents, isotonic agents, pH buffering agents, dissolution promoting agents, stabilizers, antiseptic agents and other typical auxiliary additives employed in the formulation of pharmaceutical preparations.

Optionally, the active agents and any other ingredients may be coated with an enteric coating, preferably one that is a pH sensitive polymer that dissolves at the neutral to slightly alkaline pH of the human ileum (pH 7.5). A commonly used currently approved coating of this nature is EUDRAGIT S^{™}, Rohm Pharma GmbH, Welsterstadt, Germany. The use of a pH-sensitive coating has the advantage of targeting coating dissolution to the ileum, independent of transit time. There are other forms of targeting to regions; for example, an enteric coating made of diazotized polymer may be solubilized as anaerobic bacteria in the terminal ileum and cecum, reduce and thus split the diazo bond. There are also enteric coatings, such as hydroxy cellulose, that rupture with time as they slowly hydrate and swell to bursting to provide delivery of nutrients over a longer span of the intestinal tract.

Exemplary compounds for delivery are described in U.S. Pat. Nos. 6,267,988; 5,753,253; 6,531,152; 6,429,290; 5,322,697; 6,511,975; 6,379,705; 5,668,126; 5,462,933 and U.S. Pat. Application No. 20020094346.

It is envisioned that the satiety agent catheter delivery system 12 is permanently implanted into the patient at an implantation site. It is also envisioned that the satiety agent reservoir and pump 10 could be located in the patient's abdomen or in the patient's abdominal wall. The term "implantation site" is used to refer to a site within the body of a subject at which an active agent delivery device is introduced and positioned. "Active agent delivery device or system" as used herein is meant to any implantable device suitable for delivering the formulations for management of satiety according to the invention. "Active agent delivery device" thus encompasses any implantable device with any mechanism of action including diffusive, erodible, or convective systems, *e*.*g*., osmotic pumps, biodegradable implants, electrodiffusion systems, electroosmosis systems, vapor pressure pumps, electrolytic pumps, effervescent pumps, piezoelectric pumps, erosion-based systems, or electromechanical systems. Additionally, a programmable rate pump is contemplated having a clock as an active element wherein different infusion rates are administered at different times of the day. Varying infusion rates may provide patients with a more natural appetite at pre-determined periods established in accordance with circadian rhythms.

The drug delivery device for delivery of the active agents can be implanted at the implantation site using methods and devices well known in the art. As noted above, an implantation site is a site within the body of a subject at which a drug delivery device is introduced and positioned. Implantation sites include, but are not necessarily limited to a subdermal, subcutaneous, intraperitoneal, or other suitable site within a subject's body. Subcutaneous implantation sites are preferred because of convenience in implantation and removal of the active agent delivery device. In some embodiments, the implantation site is at or near the delivery site, and thus should be a site compatible with intestinal delivery of active agent (*e*.*g*., a subcutaneous or interperitoneal site). For example, the active agent delivery device can be implanted at a subcutaneous site, and the delivery site can be a suitable site within the small intestine, preferably the ileum. Delivery of active agent from an active agent delivery device at an implantation site that is distant from a delivery site can be accomplished by providing the active agent delivery device with a catheter 20, as described herein.

In one embodiment, the implantation site can be inside the small intestine itself. In another embodiment, the implantation site can be the stomach. In yet another embodiment, there can be multiple implantation sites with multiple delivery systems 12. There can also be one or more delivery systems 12 providing a plurality of delivery catheters 20.

The delivery site is an area of the intestines to which active agent is delivered for entry into the digestive tract, *i*.*e*., a site which allows access multiple delivery systems 12. There can also be one or more delivery systems 12 providing a plurality of delivery catheters 20.

[0087] The delivery site is an area of the intestines to which active agent is delivered for entry into the digestive tract, *i.e.,* a site which allows access of the active agent to sites which cause satiety. Delivery sites include, but are not necessarily limited to, the duodenum, the caecum, the ileum or the jejunum. Ileac delivery sites are of particular interest in the present application.

A preferred form of the invention uses one or more active agents and/or electrical stimulation to treat obesity, The treatment is carried out by an implantable pump 10 and a catheter 20 having a proximal end coupled to the pump 20 and having a discharge portion for infusing therapeutic dosages of the one or more active agents into a predetermined infusion site in the small intestines. Alternatively, encapsulated cells selected to secrete the appropriate active agents or an active agent eluting polymer may be implanted into a predetermined treatment site in the small intestines. In one embodiment of the invention stimulation and/or infusion is carried out in a nearly continuous manner. In another form of the invention, the stimulation or infusion is initiated by the patient in response to hunger related symptoms or hunger provoking situations.

The present invention makes use of a system 12 wherein an active agent or other fluid to be delivered to a specific desired location within the intestine is stored in a reservoir 11 that is directly displaced by a force to infuse the active agent from the device into the patient's small intestine. Several specific methods are used to displace the reservoir, including, generally, hydraulic displacement, mechanical screw-type displacement, and spring force displacement of the fluid reservoir. Additionally, the present invention comprises a plurality of flow paths in the form of a plurality of fluidly extendable lumens or the like having differential flow rates programmable by individual valves.

A means of maintaining constant pressure in the device, such as a volatile fluid, may be necessary with an internal active agent reservoir. To refill the active agent reservoir, a syringe is introduced into the reservoir via a hypodermic needle or other suitable fluid delivery means. The active agent fluid delivery port 15 is closed and the pump is then reversed to draw the active agent from the syringe into the reservoir 11. This method also eliminates the possibility of forcing the active agent into the reservoir with the syringe and damaging the device or over-infusing the active agent.

An infusion system is disclosed that delivers active agent to the patient at a fixed rate and permits the patient to introduce a controlled bolus dosage when needed. In one example, there is a pump having a bulkhead that is provided with first and second flow paths from the pump reservoir to a single outlet port. The first flow path communicates with a first flow regulator which restricts flow in the first flow path to the desired fixed rate of delivery. The second flow path communicates with a patient-operated pumping device incorporated into the pump housing. The patient-operated pumping device may be in the form of a deformable reservoir that accumulates an active agent bolus, which may be expelled when the reservoir is compressed by the patient's fingers. A second flow regulator may be incorporated in the active agent flow path upstream of the patient-operated pumping device to restrict the dosage that may accumulate therein. A safety valve may also be incorporated into the pump housing in downstream fluid communication with the patient-operated pumping device to prevent accidental discharge of the active agent bolus.

In another preferred embodiment, a fluid control assembly may be used with single or dual port pumps. The fluid control assembly comprises a body with an inlet port and an outlet port. Two fluid control paths are defined within the body between the ports. A first fluid control path is provided with a first flow regulator for regulating a fixed flow rate of active agent between the inlet and outlet ports. A second fluid control path is in fluid communication with a patient-controlled pumping device, which accommodates a bolus of active agent that may be expelled by the patient. Both first and second fluid control paths communicate with the inlet port and outlet port. A second flow regulator is disposed in the second flow path to limit the rate of accumulation of the active agent bolus. The reservoir communicates with the flow controller outlet port via a safety valve, which prevents accidental discharge of the active agent bolus.

An infusion system is also disclosed that offers both a fixed rate of active agent delivery and the capability for a patient to deliver an active agent bolus when needed within safe dosage levels.

In general, the formulation of active compounds is delivered at a volume rate that is compatible with the delivery site, and at a dose that is therapeutically effective in induction of satiety while reducing the presence or risk of side effects.

Subjects suffering from or susceptible to obesity can receive induction of satiety for any desired period of time. In general, administration of active compounds according to the invention can be sustained release for several hours (*e*.*g*., 2 hours, 12 hours, or 24 hours to 48 hours or more), to several days (*e*.*g*., 2 to 5 days or more), to several months or years. Typically, delivery can be continued for a period ranging from about 1 month to about 12 months or more. The active compounds may be administered to an individual for a period of, for example, from about 2 hours to about 72 hours, from about 4 hours to about 36 hours, from about 12 hours to about 24 hours, from about 2 days to about 30 days, from about 5 days to about 20 days, from about 7 days or more, from about 10 days or more, from about 100 days or more, from about 1 week to about 4 weeks, from about 1 month to about 24 months, from about 2 months to about 12 months, from about 3 months to about 9 months, from about 1 month or more, from about 2 months or more, or from about 6 months or more; or other ranges of time, including incremental ranges, within these ranges, as needed. This extended period of active agent delivery is made possible by the ability of the invention to provide both adequate satiety, while minimizing the severity of any side effects. In particular embodiments, the active compounds are delivered to the subject without the need for re-accessing the device and/or without the need for re-filling the device, In these embodiments, high-concentration formulations of active compounds are of particular interest. As used herein, the terms, "sustained release" and "controlled release" indicate a prolongation of the duration of release and/or duration of action of an active agent and are well understood in the art and are intended to be interchangeable, unless otherwise indicated. herein, the terms, "sustained release" and "controlled release" indicate a prolongation of the duration of release and/or duration of action of an active agent and are well understood in the art and are intended to be interchangeable, unless otherwise indicated.

Preferably, delivery of active compounds is in a patterned fashion, more preferably in a substantially continuous fashion, *e*.*g*., substantially uninterrupted for a pre-selected period of active agent delivery, and more preferably at a substantially constant, pre-selected rate or range of rates (*e*.*g*., amount of active agent per unit time, or volume of active agent formulation for a unit time). The active agent is preferably delivered at a low volume rate of from about 1 ml/day to about 30 ml/day, preferably about 10 µl/day to about 1 ml/day, generally about 100 µl/day to about 10 ml/day, typically from about 200 µl/day to about 3.5 ml/day.

The administration of active agents by delivery using an implanted pump according to the invention is a feature of the invention. Delivery using an implanted pump is convenient for the subject, as the implantation and removal procedures are simple and can be conducted on an out-patient basis where the patient's health allows such. Subcutaneously or interperitoneally-implanted active agent delivery devices also increase patient compliance, prevent abuse, reduce the risk of infection associated with external pumps or other methods that require repeated breaking of the skin and/or maintenance of a port for administration.

Delivery of active agent to an intestinal site at a low volume rate is a particularly preferred embodiment of the invention. In general, low volume rate active agent delivery avoids accumulation of active agent at the delivery site (*e*.*g*., depot or pooling effect) by providing for a rate of administration that is less than, the same as, or only very slightly greater than the rate of removal of active agent from the delivery site.

In one embodiment, an active agent delivery device provides for substantially continuous, delivery of active agent at a preselected rate to the small intestines. For example, for intestinal delivery of active agent, the active agent can be delivered at a rate of from about 1 µg/hr to about 300 mg/hr, usually from about 1 µg/hr, 25 µg/hr, or 300 µg/hr to about 30 mg/hr, and typically between about 50 µg/hr to about 3 mg/hr. In a specific exemplary embodiment, active agent is delivered at a rate of from about 1 µg/hr, 10 µg/hr, 250 µg/hr, 1 mg/hr, generally up to about 30 mg/hr. Appropriate amounts of active compounds can be readily determined by the ordinarily skilled artisan based upon, for example, the relative potency of these active agents. The actual dose of active agent delivered will vary with a variety of factors such as the potency and other properties of the selected active agent used.

Any of a variety of controlled active agent delivery devices can be used in the present invention to accomplish delivery of an active agent formulation comprising active compounds. In general, the active agent delivery device minimally comprises a controlled active agent delivery device and, in one embodiment, further comprises an active agent delivery catheter, *e*.*g*., where the implantation site is distant from the delivery site.

Active agent delivery devices suitable for use with the present invention can take advantage of any of a variety of controlled active agent release devices. In general, the active agent release devices suitable for use in the invention comprise an active agent reservoir for retaining an active agent formulation or alternatively some substrate or matrix that can hold active agent (*e*.*g*., polymer, binding solid, *etc*.). The active agent release device can be selected from any of a variety of implantable controlled active agent delivery system known in the art. Controlled active agent release devices suitable for use in the present invention generally can provide for delivery of the active agent from the device at a selected or otherwise patterned amount and/or rate to a selected site in the subject.

In some embodiments, the delivery device is one that is adapted for delivery of active compounds over extended periods of time. Such delivery devices may be adapted for administration of active compounds for several hours (*e*.*g*., 2 hours, 12 hours, or 24 hours to 48 hours or more), to several days (*e*.*g*., 2 to 5 days or more, from about 100 days or more), to several months or years. In some of these embodiments, the device is adapted for delivery for a period ranging from about 1 month to about 12 months or more. The active agent delivery device may be one that is adapted to administer active compounds to an individual for a period of, for example, from about 2 hours to about 72 hours, from about 4 hours to about 36 hours, from about 12 hours to about 24 hours, from about 2 days to about 30 days, from about 5 days to about 20 days, from about 7 days or more, from about 10 days or more, from about 100 days or more; from about 1 week to about 4 weeks, from about 1 month to about 24 months, from about 2 months to about 12 months, from about 3 months to about 9 months, from about 1 month or more, from about 2 months or more, or from about 6 months or more; or other ranges of time, including incremental ranges, within these ranges, as needed. In these embodiments, high-concentration formulations of active compounds described herein are of particular interest for use in the invention.

Release of active agent from the system 12, particularly controlled release of active agent, can be accomplished in any of a variety of ways according to methods well known in the art. Where the active agent delivery device comprises an active agent delivery catheter 20, active agent can be delivered through the active agent delivery catheter 20 to the delivery site as a result of capillary action, as a result of pressure generated from the active agent release device or pump 10, by diffusion, by electrodiffusion or by electroosmosis through the device and/or the catheter.

The active agent delivery system 12 must be capable of carrying the active agent formulation in such quantities and concentration as therapeutically required, and must provide sufficient protection to the formulation from attack by body processes for the duration of implantation and delivery. The exterior is thus preferably made of a material that has properties to diminish the risk of leakage, cracking, breakage, or distortion so as to prevent expelling of its contents in an uncontrolled manner under stresses it would be subjected to during use, *e*.*g*., due to physical forces exerted upon the active agent release device as a result of movement by the subject or physical forces associated with pressure generated within the reservoir associated with active agent delivery. The active agent reservoir 11 or other means for holding or containing the active agent must also be of such material as to avoid unintended reactions with the active agent formulation, and is preferably biocompatible (*e*.*g*., where the device is implanted, it is substantially non-reactive with respect to a subject's body or body fluids).

Suitable materials for the reservoir or active agent holding means for use in the delivery devices of the invention are well known in the art. For example, the reservoir material may comprise a non-reactive polymer or a biocompatible metal or alloy. Suitable polymers include, but are not necessarily limited to, acrylonitrile polymers such as acrylonitrile-butadiene-styrene polymer, and the like; halogenated polymers such as polytetrafluoroethylene, polyurethane, polychlorotrifluoroethylene, copolymer tetrafiuoroethylene and hexafluoropropylene; polyethylene vinylacetate (EVA), polyimide; polysulfone; polycarbonate; polyethylene; polypropylene; polyvinylchloride-acrylic copolymer; polycarbonate-acrylonitrile-butadiene-styrene; polystyrene; cellulosic polymers; and the like. Further exemplary polymers are described in The Handbook of Common Polymers, Scott and Roff, CRC Press, Cleveland Rubber Co., Cleveland, Ohio.

Metallic materials suitable for use in the reservoir 11 of the active agent release system 12 include stainless steel, titanium, platinum, tantalum, polymers and biodegradable polymers. Exemplary biostable polymers include, but are not necessarily limited to silicone, polyurethane, polyether urethane, polyether urethane urea, polyamide, polyacetal, polyester, poly ethylene-chlorotrifluoroethylene, polytetrafluoroethylene (PTFE or "Teflon^{™}"), styrene butadiene rubber, polyethylene, polypropylene, polyphenylene oxide-polystyrene, poly-a-chloro-p-xylene, polymethylpentene, polysulfone and other related biostable polymers. Exemplary biodegradable polymers include, but are not necessarily limited to, polyanhydrides, cyclodestrans, polylactic-glycolic acid, polyorthoesters, n-vinyl alcohol, polyethylene oxide/polyethylene terephthalate, polyglycolic acid, polylactic acid and other related bioabsorbable polymers.

Where the active agent formulation is stored in a reservoir 11 comprising metal or a metal alloy, particularly titanium or a titanium alloy having greater than 60%, often greater than 85% titanium is preferred for the most size-critical applications, for high payload capability and for long duration applications and for those applications where the formulation is sensitive to body chemistry at the implantation site or where the body is sensitive to the formulation. Most preferably, the active agent delivery devices are designed for storage with active agent at room temperature or higher.

Active agent release devices suitable for use in the invention may be based on any of a variety of modes of operation. For example, the active agent release device can be based upon a diffusive system, a convective system, or an erodible system (*e*.*g*., an erosion-based system). For example, the active agent release device can be an osmotic pump, an electroosmotic pump, a vapor pressure pump, or osmotic bursting matrix, *e*.*g*., where the active agent is incorporated into a polymer and the polymer provides for release of active agent formulation concomitant with degradation of an active agent-impregnated polymeric material (*e*.*g*., a biodegradable, active agent-impregnated polymeric material). In other embodiments, the active active agent release device can be an osmotic pump, an electroosmotic pump, a vapor pressure pump, or osmotic bursting matrix, *e.g.*, where the active agent is incorporated into a polymer and the polymer provides for release of active agent formulation concomitant with degradation of an active agent-impregnated polymeric material (*e.g.*, a biodegradable, active agent-impregnated polymeric material). In other embodiments, the active agent release device is based upon an electrodiffusion system, an electrolytic pump, an effervescent pump, a piezoelectric pump, a hydrolytic system, *etc*.

Active agent release devices based upon a mechanical or electromechanical infusion pump, can also be suitable for use with the present invention. Examples of such devices include those described in, for example, U.S. Pat. Nos. 4,692,147; 4,360,019; 4,487,603; 4,360,019; 4,725,852, and the like. In general, the present methods of active agent delivery can be accomplished using any of a variety of refillable, non-exchangeable pump systems. Pumps and other convective systems are generally preferred due to their generally more consistent, controlled release over time. Osmotic pumps are particularly preferred due to their combined advantages of more consistent controlled release and relatively small size. Of the osmotic pumps, the DUROST osmotic pump is particularly preferred (see, *e*.*g*., WO 97/27840 and U.S. Pat. Nos. 5,985,305 and 5,728,396)).

In one embodiment, the active agent release device is a controlled active agent release device in the form of an osmotically-driven device. Preferred osmotically-driven active agent release systems are those that can provide for release of active agent in a range of rates of from about 10 µg/hr to about 30 mg/hr, and which can be delivered at a volume rate of from about 10 µl/day to about 30 ml/day, preferably from about 50 µl/day to about 10 ml/day, generally from about 200 µl/day to about 5 ml/day, typically from about 0.5 ml/day to about 3.5 ml/day. In one embodiment, the volume/time delivery rate is substantially constant (*e*.*g*., delivery is generally at a rate +/- about 5% to 10% of the cited volume over the cited time period, *e*.*g*., a volume rate of about.

Exemplary osmotically-driven devices suitable for use in the invention include, but are not necessarily limited to, those described in U.S. Pat. Nos. 3,760,984; 3,845,770; 3,916,899; 3,923,426; 3,987,790; 3,995,631; 3,916,899; 4,016,880; 4,036,228; 4,111,202; 4,111,203; 4,203,440; 4,203,442; 4,210,139; 4,327,725; 4,627,850; 4,865,845; 5,057,318; 5,059,423; 5,112,614; 5,137,727; 5,234,692; 5,234,693; 5,728,396; and the like.

In some embodiments it may be desirable to provide an active agent delivery catheter with the active agent delivery device, *e*.*g*., where the implantation site and the desired delivery site are not the same or adjacent. The active agent delivery catheter 20 is generally a substantially hollow elongate member having a first end (or "proximal" end) associated with the active agent release device or pump 10 of the active agent delivery system 12, and a second end (or "distal" end) for delivery of the active agent-comprising formulation to a desired delivery site within the intestines. Where an active agent delivery catheter is used, a first end of the active agent delivery catheter is associated with or attached to the active agent delivery device so that the lumen of the active agent delivery catheter is in communication with the active agent reservoir in the active agent delivery device, so that a formulation contained in an active agent reservoir can move into the active agent delivery catheter, and out a delivery outlet of the catheter which is positioned at the desired intestinal delivery site.

The body of the catheter defines a lumen, which lumen is to have a diameter compatible with providing leak-proof delivery of active agent formulation from the active agent delivery device. Where the active agent delivery device dispenses active agent by convection (as in, *e*.*g*., osmotic delivery. The distal end of the active agent delivery catheter can provide a distinct opening for delivery of active agent, or as a series of openings.

The active agent delivery catheter may be produced from any of a variety of suitable materials, and may be manufactured from the same or different material as the reservoir of the active agent release device. Impermeable materials suitable for use in production of the controlled active agent release device as described above are generally suitable for use in the production of the active agent delivery catheter. Exemplary materials from which the active agent delivery catheter can be manufactured include, but are not necessarily limited to, polymers; metals; glasses; polyolefins (high density polyethylene (HDPE), low density polyethylene (LDPE), linear low density polyethylene (LLDPE), polypropylene (PP), and the like); nylons; polyethylene terephtholate; silicones; urethanes; liquid crystal polymers; PEBAX^{™}; HYTRED^{™}; TEFLON^{™} perflouroethylene (PFE) perflouroalkoxy resins (PFA); poly(methyl methacrylate) (PMMA); multilaminates of polymer, metals, and/or glass; nitinol; and the like.

The active agent delivery catheter can comprise additional materials or agents (*e*.*g*., coatings on the external or internal catheter body surface(s)) to facilitate placement of the active agent delivery catheter and/or to provide other desirable characteristics to the catheter. For example, the active agent delivery catheter inner and/or outer walls can be coated with silver or otherwise coated or treated with antimicrobial agents, thus further reducing the risk of infection at the site of implantation and active agent delivery.

In one embodiment, the active agent delivery catheter is primed with an active agent-comprising formulation, *e*.*g*., is substantially pre-filled with active agent prior to implantation. Priming of the active agent delivery catheter reduces delivery start-up time, *i*.*e*., time related to movement of the active agent from the active agent delivery device to the distal end of the active agent delivery catheter. This feature is particularly advantageous in the present invention where the active agent release device of the active agent delivery device releases active agent at relatively low flow rates.

Methods for implanting or otherwise positioning active agent delivery devices for delivery of an active agent are well known in the art. In general, placement of the active agent delivery device will be accomplished using methods and tools that are well known in the art, and performed under aseptic conditions with at least some local or general anesthesia administered to the subject. Removal and/or replacement of active agent delivery devices can also be accomplished using tools and methods that are readily available.

The drug delivery pump may be any of a number of commercially available implantable infusion pumps such as, for example, the SYNCHROMED^{™} pump, Model 8611H, manufactured by Medtronic, Inc., Minneapolis, Minn. or the ARCHIMEDES^{™} implantable pump, Model 2350, manufactured by Codman, Germany.

U.S. Pat. No. 5,511,355 to Franetzki *et al*. discloses an infusion device intended for implantation into the human body. U.S. Pat. No. 4,969,871 to Theeuwes *et al*. discloses a drug delivery device comprising a reservoir containing a beneficial agent to be delivered. U.S. Pat. No. 4,568,331 to Fischer *et al*. discloses a disposable medicine dispensing device. Heyer Shulte Medical Catalog discloses an OMMAYA reservoir with either an on-off flushing device, or with a pressure pump. Codman Medical Catalog discloses an Ommaya reservoir with a pressure flow control. "OSMET" by Alza is a pump that works by osmotic pressure, and polyanhydride matrices by Nova.

Exemplary active agent delivery devices are described in U.S. Pat. Nos. 6,541,021; 6,464,687; 6,436,091; 6,312,409; 6,283,949; 5,836,935; 5,728,396.

The skilled artisan will appreciate that certain factors may influence the dosage required to effectively treat a subject, including but not limited to the severity of the disease or disorder, previous treatments, the general health and/or age of the subject, and other diseases present. It will also be appreciated that the effective dosage used for treatment may increase or decrease over the course of a particular treatment. Changes in dosage may result and become apparent from the results of diagnostic assays.

In addition, information regarding procedural or other details supplementary to those set forth herein is described in cited references.

## Claims

1. Use of a formulation comprising an active agent selected from the group consisting of nutrients and pharmacological by active agents in a therapeutically effective amount sufficient for inducing or promoting a feeling of satiety in a subject for a period of at least 3 days
in the manufacture of an implantable device for modulating satiety in the subject by gastro intestinal delivery of the formulation to a site of action in the subject, whereby the active agent is present at the site of action within the gastrointestinal tract in an amount sufficient to modulate satiety,
wherein the device comprises:
a controlled active agent delivery system adapted for complete implantation at an implantation site selected from the group consisting of a subcutaneous site, an intraperitoneal site, a subdermal site, an intramuscular site, and an intra-adipose tissue site in the subject; a pump and the formulation.

2. The use of claim 1, wherein the formulation comprises one or more nutrients selected from the group consisting of amino acids, peptides, proteins, lipids, carbohydrates, vitamins and minerals.

3. The use of claim 2, wherein the formulation comprises an amount of the active agent sufficient for treatment of satiety in the subject for a period of at least 3 days to 10 days.

4. The use of claim 2, wherein the formulation comprises an amount of the active agent sufficient for treatment of satiety in the subject for a period of at least 4 weeks.

5. The use of claim 2, wherein the device delivers the formulation at a rate of from 0.01 micrograms of the active agent per hour to 300 micrograms of the active agent per hour.

6. The use of claim 2, wherein the device is adapted for delivery of the formulation at a volume rate of from 0.01 microliters per day to 3 milliliters per day.

7. The use of claim 2, wherein the formulation comprises an amount of the active agent sufficient for treatment of satiety in the subject for a period of more than 7 days.

8. The use of claim 2, wherein the formulation comprises an amount of the active agent sufficient for treatment of satiety in the subject for a period of more than 20 days.

9. The use of claim 2, wherein the formulation comprises an amount of the active agent sufficient for treatment of satiety in the subject for a period of more than 30 days.

10. The use of claim 2, wherein the device further comprises a pump operably connected to housing, wherein the housing defines a reservoir and the reservoir comprises a formulation, wherein the active agent is in an amount sufficient for treatment of satiety in the subject for a period of at least 3 days, and wherein the active agent delivery system is adapted for delivery of the active agent to the ileum of a subject.

11. The use of any one of the preceding claims, wherein the device is for treating hunger or obesity in the subject.

12. The use of any one of the preceding claims, wherein the device is for intestinal delivery to the small intestine.

13. The use of any one of the preceding claims, wherein the device is for intestinal delivery to the ileum.

## Patentansprüche

1. Verwendung einer Formulierung umfassend ein aktives Agens ausgewählt aus der Gruppe bestehend aus Nährstoffen und pharmakologisch aktiven Agentien in einer therapeutisch effektiven Menge, die ausreichend ist zum Induzieren oder Fördern eines Gefühls der Sattheit in einer Person für eine Dauer von wenigstens 3 Tagen,
in der Herstellung einer implantierbaren Vorrichtung zur Modulierung von Sattheit in der Person durch Magen-Darm-Lieferung der Formulierung an eine Wirkungsstelle in der Person, wobei das aktive Agens an der Wirkungsstelle innerhalb des Magen-Darm-Trakts in einer Menge vorhanden ist, die ausreichend ist, um Sattheit zu modulieren,
wobei die Vorrichtung umfaßt:
ein gesteuertes Liefersystem für das aktive Agens, das angepaßt ist zur vollständigen Implantation an einer Implantationsstelle ausgewählt aus der Gruppe bestehend aus einer subkutanen Stelle, einer intraperitonealen Stelle, einer subdermalen Stelle, einer intramuskulären Stelle und einer Stelle zwischen dem Adiposegewebe in der Person; eine Pumpe und die Formulierung.

2. Verwendung nach Anspruch 1, wobei die Formulierung ein oder mehrere Nährstoffe umfaßt, die ausgewählt werden aus der Gruppe bestehend aus Aminosäuren, Peptiden, Proteinen, Lipiden, Kohlenhydraten, Vitaminen und Mineralien.

3. Verwendung nach Anspruch 2, wobei die Formulierung eine Menge des aktiven Agens umfaßt, die ausreichend ist zur Behandlung der Sattheit in der Person für eine Dauer von wenigstens 3 Tagen bis 10 Tagen.

4. Verwendung nach Anspruch 2, wobei die Formulierung eine Menge des aktiven Agens umfaßt, die ausreichend ist zur Behandlung der Sattheit in der Person für eine Dauer von wenigstens 4 Wochen.

5. Verwendung nach Anspruch 2, wobei die Vorrichtung die Formulierung mit einer Geschwindigkeit von 0,01 µg des aktiven Agens pro Stunde bis 300 µg des aktiven Agens pro Stunde liefert.

6. Verwendung nach Anspruch 2, wobei die Vorrichtung angepaßt ist zum Liefern der Formulierung mit einer Volumengeschwindigkeit von 0,01 µl pro Tag bis 3 ml pro Tag.

7. Verwendung nach Anspruch 2, wobei die Formulierung eine Menge des aktiven Agens umfaßt, die ausreichend ist zur Behandlung der Sattheit in der Person für eine Dauer von mehr als 7 Tagen.

8. Verwendung nach Anspruch 2, wobei die Formulierung eine Menge des aktiven Agens umfaßt, die ausreichend ist zur Behandlung der Sattheit in der Person für eine Dauer von mehr als 20 Tagen.

9. Verwendung nach Anspruch 2, wobei die Formulierung eine Menge des aktiven Agens umfaßt, die ausreichend ist zur Behandlung der Sattheit in der Person für eine Dauer von mehr als 30 Tagen.

10. Verwendung nach Anspruch 2, wobei die Vorrichtung ferner eine Pumpe umfaßt, die betriebsfähig mit einem Gehäuse verbunden ist, wobei das Gehäuse ein Reservoir definiert und das Reservoir eine Formulierung umfaßt, wobei das aktive Agens in einer Menge ist, die ausreichend ist zur Behandlung der Sattheit in der Person für eine Dauer von wenigstens 3 Tagen, und wobei das Liefersystem für das aktive Agens angepaßt ist zur Lieferung des aktiven Agens an das Ileum einer Person.

11. Verwendung nach irgendeinem der vorangehenden Ansprüche, wobei die Vorrichtung zur Behandlung von Hunger oder Fettleibigkeit in der Person ist.

12. Verwendung nach irgendeinem der vorangehenden Ansprüche, wobei die Vorrichtung zur Intestinallieferung an den Dünndarm ist.

13. Verwendung nach irgendeinem der vorangehenden Ansprüche, wobei die Vorrichtung zur Intestinallieferung an das Ileum ist.

## Revendications

1. Utilisation d'une formulation comprenant un principe actif sélectionné dans le groupe consistant en nutriments et en agents pharmacologiquement actifs en une quantité thérapeutiquement efficace suffisante pour provoquer ou favoriser une sensation de satiété chez une personne pendant une période d'au moins 3 jours dans la fabrication d'un dispositif implantable pour moduler la satiété chez le sujet par administration gastro-intestinale de la formulation vers un site d'action chez le sujet, moyennant quoi le principe actif est présent sur le site d'action dans le tractus gastro-intestinal en une quantité suffisante pour moduler la satiété, dans laquelle le dispositif comprend :
un système d'administration d'un principe actif contrôlé adapté pour achever l'implantation sur un site d'implantation sélectionné dans le groupe consistant en un site sous-cutané, un site intra-péritonéal, un site sous-dermique, un site intramusculaire, et un site tissulaire intra-adipeux chez le sujet; une pompe et la formulation.

2. Utilisation selon la revendication 1, dans laquelle la formulation comprend un ou plusieurs nutriments sélectionnés dans le groupe consistant en acides aminés, en peptides, en protéines, en lipides, en glucides, en vitamines et en minéraux.

3. Utilisation selon la revendication 2, dans laquelle la formulation comprend une quantité du principe actif suffisante pour le traitement de la satiété chez le sujet pendant une période d'au moins 3 à 10 jours.

4. Utilisation selon la revendication 2, dans laquelle la formulation comprend une quantité du principe actif suffisante pour le traitement de la satiété chez le sujet pendant une période d'au moins 4 semaines.

5. Utilisation selon la revendication 2, dans laquelle le dispositif administre la formulation à un rythme compris entre 0,01 microgramme de principe actif par heure et 300 microgrammes de principe actif par heure.

6. Utilisation selon la revendication 2, dans laquelle le dispositif est adapté pour l'administration de la formulation à un débit compris entre 0,01 microlitre par jour et jusqu'à 3 millilitres par jour.

7. Utilisation selon la revendication 2, dans laquelle la formulation comprend une quantité du principe actif suffisante pour le traitement de la satiété chez le sujet pendant une période de plus de 7 jours.

8. Utilisation selon la revendication 2, dans laquelle la formulation comprend une quantité de l'agent actif suffisante pour le traitement de la satiété chez le sujet pendant une période de plus de 20 jours.

9. Utilisation selon la revendication 2, dans laquelle la formulation comprend une quantité de l'agent actif suffisante pour le traitement de la satiété chez le sujet pendant une période de plus de 30 jours.

10. Utilisation selon la revendication 2, dans laquelle le dispositif comprend en outre une pompe reliée de manière opérationnelle à un logement, dans laquelle le logement définit un réservoir et le réservoir comprend une formulation, dans laquelle le principe actif se trouve en une quantité suffisante pour le traitement de la satiété chez le sujet pendant une période d'au moins 3 jours, et dans laquelle le système d'administration du principe actif est adapté pour l'administration du principe actif vers l'iléon d'un sujet.

11. Utilisation selon l'une quelconque des revendications précédentes, dans laquelle le dispositif est destiné au traitement de la faim ou de l'obésité chez le sujet.

12. Utilisation selon l'une quelconque des revendications précédentes, dans laquelle le dispositif est destiné à une administration intestinale vers l'intestin grêle.

13. Utilisation selon l'une quelconque des revendications précédentes, dans laquelle le dispositif est destiné à une administration intestinale vers l'iléon.
